# EUROPÄISCHE PATENTSCHRIFT

(11) **EP 3 784 211 B1**
(45) Veröffentlichungstag und Bekanntmachung des Hinweises auf die Patenterteilung: **20.07.2022**
(21) Anmeldenummer: 19719882.3
(22) Anmeldetag: 25.04.2019
(51) Int. Cl.: A61K 9/00, A61K 31/135, A61K 47/32, A61K 47/38, A61K 47/34, A61K 47/36

(54) **U-FÖRMIGER ORALER DÜNNFILM**
U-SHAPED ORAL THIN FILM
FILM MINCE BUCCO-DENTAIRE EN FORME DE U

(30) Priorität: 25.04.2018 DE 102018109981
(43) Veröffentlichungstag der Anmeldung: 03.03.2021
(73) Patentinhaber: LTS Lohmann Therapie-Systeme AG, 56626 Andernach (DE)
(72) Erfinder: SCHMITZ, Christoph, 56598 Rheinbrohl (DE); BAUER, Marius, 56626 Andernach (DE); LINN, Michael, 55596 Waldböckelheim (DE)
(74) Vertreter: Held, Stephan
(86) Internationale Anmeldenummer: PCT/EP2019/060665
(87) Internationale Veröffentlichungsnummer: WO 2019/207067

(56) Entgegenhaltungen:
- US-A1- 2014 010 851
- Vrbata Et Al: "Electrospun drug loaded membranes for sublingual administration of sumatriptan and naproxen", INTERNATIONAL JOURNAL OF PHARMACEUTICS, 30. November 2013 (2013-11-30), XP55599701, Gefunden im Internet: URL:https://www.sciencedirect.com/science/ article/pii/S0378517313008193#fig0080 [gefunden am 2019-06-26]

## Beschreibung

Die vorliegende Erfindung betrifft einen U-förmigen oralen Dünnfilm und den U-förmigen oralen Dünnfilm zur Verwendung als Arzneimittel, insbesondere zur sublingualen Applikation.

Orale Dünnfilme sind dünne, mindestens einen pharmazeutisch aktiven Wirkstoff enthaltende Filme, die direkt in den Mundraum gelegt oder an die Mundschleimhaut angelegt werden und sich dort auflösen. Dabei handelt es sich insbesondere um dünne wirkstoffhaltige Filme auf Polymerbasis, die, wenn sie auf eine Schleimhaut, insbesondere die Mundschleimhaut, aufgebracht werden, den Wirkstoff direkt in diese abgeben. Insbesondere kann der orale Dünnfilm in den Raum bzw. auf die Schleimhaut unter der Zunge appliziert werden (sublinguale Applikation). Aufgrund der guten Gefäßversorgung und der starken Durchblutung der Mundschleimhaut in diesem Bereich, findet eine rasche Resorption des Wirkstoffs statt.

Diese Darreichungsform hat den Vorteil, dass der Wirkstoff größtenteils durch die Schleimhaut resorbiert wird und damit der "First-Pass-Metabolismus", der bei der konventionellen Darreichungsform eines Wirkstoffs in Tablettenform auftritt, vermieden wird. Der Wirkstoff kann dabei in dem Film gelöst, emulgiert oder dispergiert werden.

Die aus dem Stand der Technik bekannten oralen Dünnfilme, auch die zur sublingualen Applikation, sind fast ausschließlich von rechteckiger Form, da diese Form am einfachsten und kostengünstigsten herzustellen ist. Es sind auch orale Dünnfilme zur sublingualen Applikation bekannt, die im Wesentlichen rechteckig sind, jedoch eine kleine Aussparung für die Aufnahme des "Frenulum linguae" ("Zungenbändchens") aufweisen, um den Tragekomfort zu erhöhen.

So offenbart die US 8,475,832 A1 einen rechteckigen oralen Dünnfilm umfassend Buprenorphin als aktiven Wirkstoff, u.a. zur sublingualen Applikation.

Die WO 2013/173697 A1 offenbart orale Dünnfilme in Streifenform zur Verwendung in der Therapie von Allergien.

Die US 2014/0010851 offenbart einen oralen Dünnfilm zur Zahnpflege, umfassend ein Polymer und einen Wirkstoff, wobei der orale Dünnfilm im Wesentlichen U-förmig ist.

Die Publikation Vrbata ez al: "Electrospun drug loaded membranes for sublingual administration of sumatrapin and naproxen" im International Journal of Pharmaceutics vom 30. November 2013 offenbart orale Dünnfilme, die ein Polymer sowie Sumatripan und Naproxen als Wirkstoffe umfassen, und wobei der orale Dünnfilm eine U-förmige Membran darstellt.

Diese aus dem Stand der Technik bekannten oralen Dünnfilme haben den Nachteil, dass der verfügbare Raum unter der Zunge nicht optimal ausgenutzt wird. Dies ist insbesondere nachteilig bei der Verabreichung von Wirkstoffen, die in einer relativ hohen Dosis sublingual verabreicht werden sollen. Da das Flächengewicht und damit die Wirkstoffmenge pro Flächeneinheit in einem oralen Dünnfilm durch viele Faktoren begrenzt ist (u.a. Trocknungszeit, Auflösungszeit des oralen Dünnfilms etc.) ist es daher nötig, den zur Verfügung stehenden Raum bestmöglich zu nutzen. Dies ist bei im Wesentlichen rechteckigen oralen Dünnfilmen bei der sublingualen Applikation nicht der Fall.

Die Aufgabe der vorliegenden Erfindung besteht darin, vorstehend genannte Nachteile des Standes der Technik zu beheben. Insbesondere besteht die Aufgabe der vorliegenden Erfindung darin, einen oralen Dünnfilm bereitzustellen, der den Raum unter der Zunge optimal ausnutzt, um insbesondere auch große Wirkstoffmengen zu applizieren. Zudem soll der orale Dünnfilm auch kostengünstig, d.h. ohne viel Materialverlust herstellbar sein.

Obige Aufgabe wird durch einen oralen Dünnfilm gemäß Anspruch 1 gelöst, d. h. durch einen oralen Dünnfilm umfassend mindestens ein Polymer und mindestens einen pharmazeutisch aktiven Wirkstoff, dadurch gekennzeichnet, dass der orale Dünnfilm im Wesentlichen U-förmig ist und wobei die beiden Enden (3) des U-förmigen oralen Dünnfilms im Wesentlichen parallel verlaufen, wobei der mindestens eine pharmazeutisch aktive Wirkstoff Ketamin in einer Menge von 20 bis 40 Gew.-% umfasst und wobei das mindestens eine Polymer Hydroxypropylmethylcellulose oder Polyvinylalkohol umfasst..

Ein solcher U-förmiger oraler Dünnfilm zeichnet sich dadurch aus, dass er den zur Verfügung stehenden Raum unter der Zunge optimal ausnutzt. Insbesondere durch die Schenkel des U-förmigen oralen Dünnfilms wird der Raum unter der Zunge von dem Bereich der vorderen Schneidezähne bis nach hinten in den Bereich der Zungenwurzel optimal genutzt. Zudem weist ein solcher U-förmiger oraler Dünnfilm einen hohen Tragekomfort auf, da zum einen das Frenulum linguae ausgespart ist und zum anderen keine scharfen Ecken oder Spitzen vorhanden sind, die den Tragekomfort negativ beeinflussen könnten. Außerdem hat ein solcher U-förmiger oraler Dünnfilm den Vorteil, dass er bei entsprechender Anordnung ohne viel Verluste aus einem großflächigen Film ausstanzbar oder ausschneidbar ist.

### Beschreibung der Figuren

Figur 1:

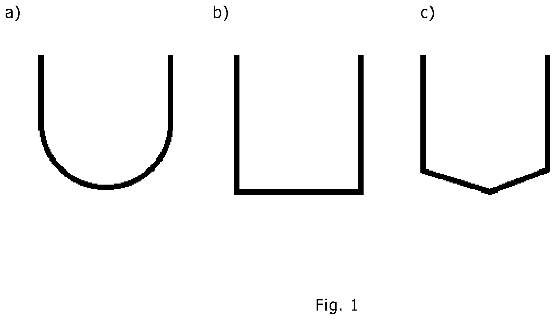

   Figur 1 zeigt eine schematische Darstellung von Formen, die unter den erfindungsgemäß gebrauchten Begriff "U förmig" subsumiert werden. Diese stellen lediglich Grenzformen dar, sodass alle dazwischenliegenden Formen ebenfalls unter den Begriff "U förmig" fallen.
Figur 2:

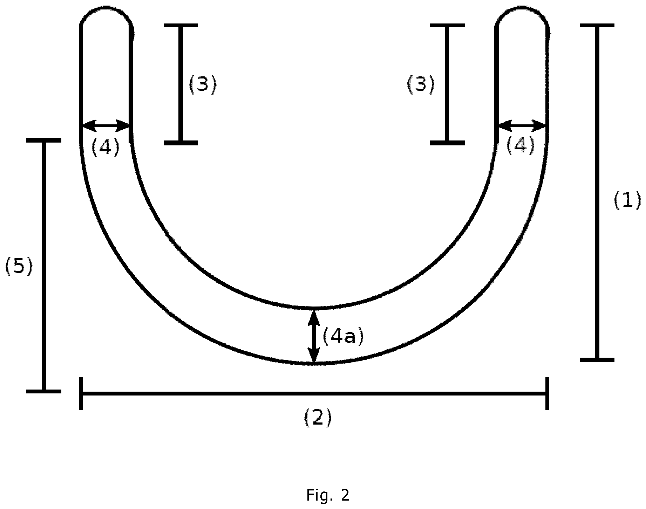

   Figur 2 zeigt eine schematische Darstellung eines erfindungsgemäßen U-förmigen oralen Dünnfilms.
   (1) Gesamtlänge des oralen Dünnfilms
   (2) Gesamtbreite des oralen Dünnfilms
   (3) Länge der im Wesentlichen parallelen Enden
   (4) Breite des oralen Dünnfilms im Bereich der im wesentlichen parallelen Enden
      (4a) Breite des oralen Dünnfilms im Bereich der Verbindung
   (5) Länge des oralen Dünnfilms im Bereich der Verbindung
Figur 3:

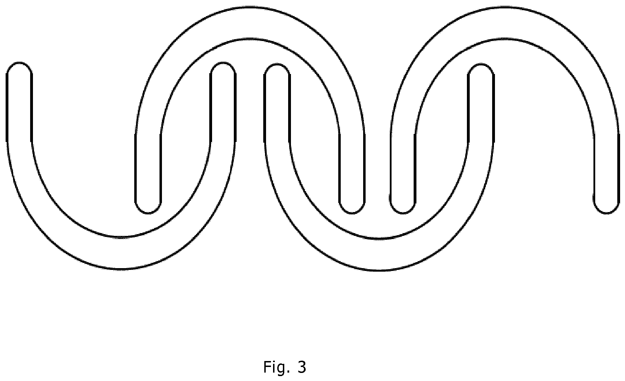

   Figur 3 zeigt eine Möglichkeit, wie erfindungsgemäße U-förmige orale Dünnfilme trotz ihrer relativ komplexen Form ohne große Materialverluste aus einer Vorlage ausgeschnitten oder ausgestanzt werden können.

Der erfindungsgemäße orale Dünnfilm umfasst mindestens ein Polymer, wobei das mindestens eine Polymer Hydroxypropylmethylcellulose oder Polyvinylalkohol umfasst.

Der erfindungsgemäße orale Dünnfilm umfasst zudem mindestens einen pharmazeutisch aktiven Wirkstoff, wobei der mindestens eine pharmazeutisch aktive Wirkstoff Ketamin umfasst.

Der erfindungsgemäße orale Dünnfilm zeichnet sich insbesondere dadurch aus, dass er im Wesentlichen U-förmig ist. Unter U-förmig wird verstanden, dass der orale Dünnfilm im Wesentlichen dem Buchstaben "U" des lateinischen Alphabets ähnelt.

Unter U-förmig wird demnach eine Form verstanden, wobei zwei im Wesentlichen parallele Enden an jeweils einem Ende auf derselben Seite durch eine Verbindung miteinander verbunden werden. Diese Verbindung kann durch eine gebogene Verbindung ermöglicht werden, sodass ein "rundes" U gebildet wird. Diese Verdingung kann jedoch auch durch eine gerade Verbindung gebildet werden, sodass ein eckiges " 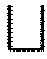 " gebildet wird.

Ferner sind auch Ausführungsformen des erfindungsgemäßen oralen Dünnfilms unter "U förmig" zu subsumieren, bei denen die Verbindung der beiden im wesentlichen parallelen Enden auf jeweils derselben Seite durch zwei Schenkel, die in einer Spitze zusammenlaufen, also einer Art Dreieck ohne Basis, gebildet wird. Es entsteht ein "spitzes eckiges " 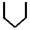 ".

Beispiele für Formen, wie sie oben beschreiben sind und die unter den Begriff U-förmig fallen, sind in der Figur 1 dargestellt.

Konkret wird in Figur 1a das "runde" U, in Figur 1b das "eckige" U und in Figur 1c das "spitze eckige" U dargestellt.

Diese konkret beschriebenen Formen stellen jedoch lediglich Grenzformen dar, sodass alle dazwischenliegenden Formen ebenfalls unter den Begriff "U förmig" fallen.

Im Folgenden werden bevorzugte Ausführungsformen des erfindungsgemäßen "U-förmigen" oralen Dünnfilms erwähnt.

Die im Folgenden dargestellten Dimensionen mit den zugehörigen Bezugszeichen beziehen sich auf die Ausführungsform des "runden U". Diese sind jedoch auch analog auf die anderen definierten Formen, wie das "eckige U" und das "spitze eckige U" anzuwenden.

Der U-förmige orale Dünnfilm weist einen gebogenen Bereich über die Gesamtbreite (2) des oralen Dünnfilms, der sich auch auf einen Teil (5) der Gesamtlänge (1) des oralen Dünnfilms erstreckt, sowie zwei jeweils an die Verbindungen anschließende Enden auf. Die Verbindung (2),(5) des oralen Dünnfilms ist so gestaltet, dass auslaufenden Enden (3) im Wesentlichen parallel sind.

Der erfindungsgemäße orale Dünnfilm ist dadurch gekennzeichnet, dass die beiden Enden (3) im Wesentlichen parallel zueinander verlaufen. Dabei verlaufen vorzugsweise alle vier Kanten, d. h. die beiden Innenkanten sowie die beiden Außenkanten des oralen Dünnfilms, im Wesentlichen parallel zueinander.

Unter im Wesentlichen parallel wird verstanden, dass die Kanten von 0° bis etwa 15° von einer gedachten exakt parallelen Anordnung in beide Richtungen in der Flächenebene abweichen können.

Die im Wesentliche parallele Anordnung der Enden (3) hat den Vorteil, dass auch die Räume im hinteren Bereich unter der Zunge, also hin zur Zungenwurzel optimal ausgenutzt werden können.

Vorzugsweise sind die Ecken der Enden (3) leicht abgerundet, sodass der Tragekomfort erhöht wird.

Der erfindungsgemäße orale Dünnfilm ist außerdem vorzugsweise dadurch gekennzeichnet, dass dieser eine im Wesentlichen konstante Breite (4),(4a) im Bereich der im Wesentlichen parallelen Enden und im Bereich der Verbindung aufweist.

Unter der Breite (4) wird die Breite der tatsächlichen Oberfläche des oralen Dünnfilms im Bereich der parallelen Enden verstanden. Unter der Breite (4a) wird die Breite der tatsächlichen Oberfläche des oralen Dünnfilms im Bereich der Verbindung (2),(5), die durch die Gesamtbreite des oralen Dünnfilms (2) und die Länge der Verbindung (5) definiert wird, verstanden.

Die im Wesentlichen konstante Breite (4),(4a) im Bereich der im Wesentlichen parallelen Enden und im Bereich der Verbindung beträgt vorzugsweise etwa 0,6 bis 1,4 cm.

Eine im Wesentlichen konstante Breite (4),(4a) hat den Vorteil, dass der orale Dünnfilm sich gleichmäßig auflöst und somit den Wirkstoff konstant abgibt.

Der erfindungsgemäße orale Dünnfilm ist weiterhin vorzugsweise dadurch gekennzeichnet, dass dieser eine Gesamtlänge (1) von etwa 2,0 bis 4,0 cm aufweist.

Der orale Dünnfilm zeichnet sich vorzugsweise außerdem dadurch aus, dass dieser eine Gesamtbreite (2) von etwa 3,0 bis 4,5 cm aufweist.

In einigen Ausführungsformen ist es bevorzugt, dass die Breite (4a) im Bereich der Verbindung (2),(5) des erfindungsgemäßen oralen Dünnfilms etwas größer ist als die Breite (4) im Bereich der in Wesentlichen parallelen Enden (3). Dabei ist es bevorzugt, dass der orale Dünnfilm im Bereich der Verbindung (2),(5) eine um 0,01 bis 20% größere Breite (4a) als die Breite (4) im Bereich der im Wesentlichen parallelen Enden (3) aufweist.

Dies hat den Vorteil, dass der Raum unter der Zunge noch besser ausgenutzt werden kann, was insbesondere bei der Verabreichung von relativ großen Wirkstoffmengen vorteilhaft ist.

Die Gesamtlänge (1) ist die Summe der Länge der Verbindung (5) und der Länge der im Wesentlichen parallelen Enden (3). Die Länge der Verbindung (5) und die Länge der im Wesentlichen parallelen Enden (3) können unabhängig voneinander eingestellt werden, um orale Dünnfilme bereitzustellen, die den unterschiedlichen Kiefergrößen und Kieferformen der Patienten Rechnung tragen.

Der erfindungsgemäße orale Dünnfilm ist vorzugsweise dadurch gekennzeichnet, dass die Verbindung (5) eine Gesamtlänge von etwa 1,5 bis 2,4 cm aufweist.

Der erfindungsgemäße orale Dünnfilm ist weiterhin vorzugsweise dadurch gekennzeichnet, dass die im Wesentlichen parallelen Enden (3) jeweils eine Gesamtlänge von etwa 0,1 bis 1,5 cm aufweisen.

Der erfindungsgemäße orale Dünnfilm ist außerdem vorzugsweise dadurch gekennzeichnet, dass die Gesamtfläche einer Oberfläche des erfindungsgemäßen oralen Dünnfilms 1 bis 10 cm² beträgt.

Bevorzugt weist der erfindungsgemäße orale Dünnfilm eine Dicke von bis zu 1 mm auf. Es sind jedoch auch Ausführungsformen des erfindungsgemäßen oralen Dünnfilm möglich, bei denen der erfindungsgemäße orale Dünnfilm eine Dicke von bis zu 3 mm aufweist.

Der erfindungsgemäße orale Dünnfilm ist vorzugsweise zur Verabreichung an erwachsene Patienten konzipiert. Sollte ein solcher oraler Dünnfilm zur Applikation bei Babys oder Kindern bereitgestellt werden, so muss die Größe des oralen Dünnfilms, d.h. die Gesamtlänge (1), die Gesamtbreite (2), die Länge der Verbindung (5) und die Länge der im Wesentlichen parallelen Enden (3), sowie die Breite (4) und (4a) an die Kiefergrößen der Babys oder Kinder angepasst werden, was dem Fachmann ohne weiteres möglich ist.

Es ist bevorzugt, dass bei einem erfindungsgemäßen orale Dünnfilm in U-Form, insbesondere bei dem "eckigen" und dem "eckigen spitzen" U, die Kanten und Ecken jeweils abgerundet sind.

Besonders bevorzugt ist der erfindungsgemäße orale Dünnfilm in Form des "runden U", da dieser die wenigsten scharfen Kanten und/oder Ecken aufweist.

Der erfindungsgemäße orale Dünnfilm umfasst ein Polymer, wobei das Polymer Hydroxypropylcellulose oder Polyvinylalkohol, umfasst. Das Polymer ist vorzugsweise ein wasserlösliches und/oder wasserquellbares Polymer.

Wasserlösliche und/oder wasserquellbare Polymere umfassen chemisch sehr unterschiedliche, natürliche oder synthetische Polymere, deren gemeinsames Merkmal ihre Löslichkeit bzw. Quellbarkeit in Wasser oder wässrigen Medien ist.

Das Flächengewicht des oralen Dünnfilms beträgt vorzugsweise etwa 20 bis 300 g/m², besonders bevorzugt etwa 100 bis 250 g/m².

Die Menge von Wirkstoff in dem erfindungsgemäßen oralen Dünnfilm hängt von dessen Art ab und beträgt üblicherweise etwa 0,01 und 70 Gew.-%.

Bevorzugt umfasst der erfindungsgemäße orale Dünnfilm außerdem noch mindestens einen Hilfsstoff ausgewählt aus der Gruppe umfassend Farbstoffe, Aromastoffe, Süßstoffe, geschmacksmaskierende Mittel, Tenside, Enhancer, pH-Regulatoren, Konservierungsmittel, Antioxidationsmittel und/oder Weichmacher.

Diese Hilfsstoffe sind dabei vorzugsweise jeweils in einer Menge von 0,001 bis 20 Gew.-% in dem erfindungsgemäßen oralen Dünnfilm enthalten.

Der erfindungsgemäße orale Dünnfilm ist vorzugsweise ein einschichtiger U-förmiger oraler Dünnfilm. Es sind aber auch Ausführungsformen möglich, in denen der erfindungsgemäße orale Dünnfilm ein mehrschichtiger U-förmiger oraler Dünnfilm ist. Die einzelnen Schichten können mittels Aufeinanderbeschichten, Auflaminieren, Siegelungen oder Klebstoffe miteinander verbunden werden. In solchen mehrschichtigen oralen Dünnfilm kann jede einzelne Schicht denselben oder verschiedene pharmazeutisch aktive Wirkstoffe enthalten.

Die vorliegende Erfindung betrifft außerdem den erfindungsgemäßen oralen Dünnfilm zur Verwendung als Arzneimittel, insbesondere zur sublingualen Applikation.

Der erfindungsgemäße orale Dünnfilm kann gemäß den üblichen Verfahren zur Herstellung von oralen Dünnfilmen hergestellt werden. Beispielweise umfasst ein solches Verfahren die Schritte
a) Herstellen einer Suspension oder Lösung, umfassend das mindestens eine Polymer und den mindestens einen pharmazeutisch aktiven Wirkstoff;
b) Ausstreichen und Trocknen der in Schritt a) erhaltenen Suspension oder Lösung, sodass ein Dünnfilm, vorzugsweise mit einem Flächengewicht von 20 bis 350 g/m² erhalten wird; und
c) Ausschneiden oder Ausstanzen des U -förmigen oralen Dünnfilms aus dem Dünnfilm, der in Schritt b) erhalten wurde.

Die U-Form des erfindungsgemäßen oralen Dünnfilms hat den Vorteil, dass dieser trotz der relativ komplexen Form ohne viel Verluste aus dem Dünnfilm in Schritt c) des oben beschriebenen Verfahrens ausgeschnitten oder ausgestanzt werden kann. Dies ist schematisch für vier U-förmige orale Dünnfilme in Form des "runden" U in Figur 3 dargestellt.

## Patentansprüche

1. Oraler Dünnfilm umfassend mindestens ein Polymer und mindestens einen pharmazeutisch aktiven Wirkstoff, **dadurch gekennzeichnet, dass** der orale Dünnfilm im Wesentlichen U-förmig ist und wobei die beiden Enden (3) des U-förmigen oralen Dünnfilms im Wesentlichen parallel verlaufen, wobei der mindestens eine pharmazeutisch aktive Wirkstoff Ketamin in einer Menge von 20 bis 40 Gew.-% umfasst und wobei das mindestens eine Polymer Hydroxypropylmethylcellulose oder Polyvinylalkohol umfasst.

2. Oraler Dünnfilm gemäß irgendeinem der vorhergehenden Ansprüche, **dadurch gekennzeichnet, dass** der orale Dünnfilm eine im Wesentlichen konstante Breite (4),(4a) im Bereich der im Wesentlichen parallelen Enden (3) und im Bereich der Verbindung (2),(5), vorzugsweise eine konstante Breite von 0,6 bis 1,4 cm aufweist.

3. Oraler Dünnfilm gemäß irgendeinem der vorhergehenden Ansprüche, **dadurch gekennzeichnet, dass** der orale Dünnfilm eine Gesamtlänge (1) von 2,0 bis 4,0 cm aufweist.

4. Oraler Dünnfilm gemäß irgendeinem der vorhergehenden Ansprüche, **dadurch gekennzeichnet, dass** der orale Dünnfilm eine Gesamtbreite (2) von 3,0 bis 4,5 cm aufweist.

5. Oraler Dünnfilm gemäß irgendeinem der vorhergehenden Ansprüche, **dadurch gekennzeichnet, dass** der orale Dünnfilm im Bereich der Verbindung (2),(5) eine um 0,01 - 20 % größere Breite (4a) als die Breite (4) im Bereich der im Wesentlichen parallelen Enden aufweist.

6. Oraler Dünnfilm gemäß irgendeinem der vorhergehenden Ansprüche, **dadurch gekennzeichnet, dass** die Verbindung (5) eine Gesamtlänge von 1,5 bis 2,4 cm aufweist.

7. Oraler Dünnfilm gemäß irgendeinem der vorhergehenden Ansprüche, **dadurch gekennzeichnet** das die im Wesentlichen parallelen Enden (3) jeweils eine Gesamtlänge von 0,1 bis 1,5 cm aufweisen.

8. Oraler Dünnfilm gemäß irgendeinem der vorhergehenden Ansprüche, **dadurch gekennzeichnet, dass** die Gesamtfläche einer Oberfläche des oralen Dünnfilms 1 bis 10 cm² beträgt.

9. Oraler Dünnfilm gemäß irgendeinem der vorhergehenden Ansprüche, **dadurch gekennzeichnet, dass** der orale Dünnfilm ein Flächengewicht von 20 bis 350 g/m² aufweist.

10. Oraler Dünnfilm gemäß irgendeinem der vorhergehenden Ansprüche, **dadurch gekennzeichnet, dass** der orale Dünnfilm mindestens einen Hilfsstoff ausgewählt aus der Gruppe, umfassend Farbstoffe, Aromastoffe, Süßstoffe, geschmacksmaskierende Mittel, Tenside, Enhancer, pH-Regulatoren, Konservierungsmittel, Antioxidationsmittel und/oder Weichmacher, enthält.

11. Oraler Dünnfilm gemäß irgendeinem der Ansprüche 1 bis 10 zur Verwendung als Arzneimittel.

12. Oraler Dünnfilm gemäß irgendeinem der Ansprüche 1 bis 10 zur Verwendung als Arzneimittel zur sublingualen Applikation.

## Claims

1. Oral thin film comprising at least one polymer and at least one pharmaceutically active ingredient, **characterised in that** the oral thin film is substantially U-shaped and wherein the oral thin film is constructed in such a way that the two ends (3) are substantially parallel, wherein the at least one pharmaceutically active ingredient comprises ketamine in a quantity of 20 to 40% by weight and wherein the at least one polymer comprises hydroxypropyl methylcellulose or polyvinyl alcohol.

2. Oral thin film according to either one of the preceding claims, **characterised in that** the oral thin film has a substantially constant width (4), (4a) in the region of the substantially parallel ends (3) and, in the region of the join (2), (5), preferably has a constant width of from 0.6 to 1.4 cm.

3. Oral thin film according to any one of the preceding claims, **characterised in that** the oral thin film has a total length (1) of from 2.0 to 4.0 cm.

4. Oral thin film according to any one of the preceding claims, **characterised in that** the oral thin film has a total width (2) of from 3.0 to 4.5 cm.

5. Oral thin film according to any one of the preceding claims, **characterised in that** the oral thin film has a width (4a) in the region of the join (2), (5) which is 0.01 - 20% greater than the width (4) in the region of the substantially parallel ends.

6. Oral thin film according to any one of the preceding claims, **characterised in that** the join (5) has a total length of from 1.5 to 2.4 cm.

7. Oral thin film according to any one of the preceding claims, **characterised in that** the substantially parallel ends (3) each have a total length of from 0.1 to 1.5 cm.

8. Oral thin film according to any of the preceding claims, **characterised in that** the total area of a surface of the oral thin film is 1 to 10 cm².

9. Oral thin film according to any one of the preceding claims, **characterised in that** the oral thin film has a weight per unit area of from 20 to 350 g/m².

10. Oral thin film according to any of the preceding claims, **characterised in that** the oral thin film contains at least one auxiliary selected from the group comprising dyes, flavourings, sweeteners, taste-masking agents, surfactants, enhancers, pH regulators, preservatives, antioxidants and/or plasticisers.

11. Oral thin film according to any one of claims 1 to 10 for use as a medicament.

12. Oral thin film according to any one of claims 1 to 10 for use as a medicament for sublingual application.

## Revendications

1. Film mince bucco-dentaire comprenant au moins un polymère et au moins un principe actif pharmaceutique, **caractérisé en ce que** le film mince bucco-dentaire est sensiblement en forme de U et dans lequel les deux extrémités (3) du film mince bucco-dentaire en forme de U s'étendent sensiblement parallèlement, dans lequel le au moins un principe actif pharmaceutique comprend de la kétamine dans une quantité de 20 à 40 % en poids et dans lequel le au moins un polymère comprend de l'hydroxypropylméthylcellulose ou de l'alcool polyvinylique.

2. Film mince bucco-dentaire selon l'une quelconque des revendications précédentes, **caractérisé en ce que** le film mince bucco-dentaire présente une largeur (4), (4a) sensiblement constante dans la zone des extrémités (3) sensiblement parallèles et dans la zone de la liaison (2), (5), de préférence une largeur constante de 0,6 à 1,4 cm.

3. Film mince bucco-dentaire selon l'une quelconque des revendications précédentes, **caractérisé en ce que** le film mince bucco-dentaire présente une longueur totale (1) de 2,0 à 4,0 cm.

4. Film mince bucco-dentaire selon l'une quelconque des revendications précédentes, **caractérisé en ce que** le film mince bucco-dentaire présente une largeur totale (2) de 3,0 à 4,5 cm.

5. Film mince bucco-dentaire selon l'une quelconque des revendications précédentes, **caractérisé en ce que** le film mince bucco-dentaire présente dans la zone de la liaison (2), (5) une largeur (4a) de 0,01-20 % supérieure à la largeur (4) dans la zone des extrémités sensiblement parallèles.

6. Film mince bucco-dentaire selon l'une quelconque des revendications précédentes, **caractérisé en ce que** la liaison (5) présente une longueur totale de 1,5 à 2,4 cm.

7. Film mince bucco-dentaire selon l'une quelconque des revendications précédentes, **caractérisé en ce que** les extrémités (3) sensiblement parallèles présentent respectivement une longueur totale de 0,1 à 1,5 cm.

8. Film mince bucco-dentaire selon l'une quelconque des revendications précédentes, **caractérisé en ce que** la surface totale d'une surface du film mince bucco-dentaire atteint 1 à 10 cm².

9. Film mince bucco-dentaire selon l'une quelconque des revendications précédentes, **caractérisé en ce que** le film mince bucco-dentaire présente une masse surfacique de 20 à 350 g/m².

10. Film mince bucco-dentaire selon l'une quelconque des revendications précédentes, **caractérisé en ce que** le film mince bucco-dentaire contient au moins un adjuvant sélectionné dans le groupe comprenant des colorants, substances aromatisantes, édulcorants, agents de masquage de goût, tensioactifs, exhausteurs, régulateurs de pH, conservateurs, antioxydants et/ou plastifiants.

11. Film mince bucco-dentaire selon l'une quelconque des revendications 1 à 10 pour son utilisation comme médicament.

12. Film mince bucco-dentaire selon l'une quelconque des revendications 1 à 10 pour son utilisation comme médicament pour application sublinguale.
